# EUROPÄISCHE PATENTSCHRIFT

(11) **EP 1 993 644 B1**
(45) Veröffentlichungstag und Bekanntmachung des Hinweises auf die Patenterteilung: **23.05.2012**
(21) Anmeldenummer: 07726709.4
(22) Anmeldetag: 08.03.2007
(51) Int. Cl.: A61M 15/00

(54) **INHALATOR FÜR PULVERFÖRMIGE SUBSTANZEN**
INHALATOR FOR POWDERY SUBSTANCES
INHALATEUR POUR SUBSTANCES PULVÉRULENTES

(30) Priorität: 10.03.2006 DE 102006011559; 27.06.2006 DE 102006029753
(43) Veröffentlichungstag der Anmeldung: 26.11.2008
(73) Patentinhaber: Von Schuckmann, Alfred, 47627 Kevelaer (DE)
(72) Erfinder: Von Schuckmann, Alfred, 47627 Kevelaer (DE)
(74) Vertreter: Rieder, Hans-Joachim
(86) Internationale Anmeldenummer: PCT/EP2007/052172
(87) Internationale Veröffentlichungsnummer: WO 2007/104694

(56) Entgegenhaltungen:
- WO-A-00/64519
- WO-A-01/21238
- WO-A-93/16748
- WO-A-2005/016424

## Beschreibung

Die Erfindung betrifft einen Inhalator für pulverförmige Substanzen, insbesondere medizinische Substanzen, mit einer Substanzen-Vorratskammer und einer eine bestimmte Menge davon aufnehmenden Dosierkammer, welche als Querbohrung eines Stabes ausgebildet und von einer Befüllstellung in eine Entleerungsstellung verschiebbar ist, in welcher Entleerungsstellung die Dosierkammer in einem Luftströmungskanal liegt.

Inhalatoren der in Rede stehenden Art sind bekannt. So wird beispielsweise auf die US 5,239,992 verwiesen. In dieser Patentschrift ist ein Inhalator dargestellt und beschrieben, der einen, eine Dosierkammer aufweisenden Stab besitzt, der von einer Befüllstellung in eine Entleerungsstellung verschiebbar ist. Diese Verlagerung in die Entleerungsstellung erfolgt durch eine Bewegung, die auf den Unterdruck zurückzuführen ist, welcher von der Einatmung des Patienten durch den Luftströmungskanal erzeugt wird. Dosierkammerverlagerung und Entleerung der Dosierkammer ist entsprechend abhängig von einer Ansaugung durch den Patienten im Zuge eines tiefen Einatmens.

Dokument WO01/21238 offenbart einen Inhalator mit einer Substanzen-Vorratskammer und einer Dosierkammer welche als Querbohrung eines Flachstabes ausgebildet und von einer Befüllungstellung in eine Entleerungsstellung verschiebbar ist, in welcher Entleereungsstellung die Dosierkammer in einem Luftströmungskanal liegt.

Im Hinblick auf den bekannten Stand der Technik stellt sich der Erfindung die Aufgabe, einen gattungsgemäßen Inhalator insbesondere im Hinblick auf die Entleerung der Dosierkammer in der Entleerungsstellung in vorteilhafter Weise weiter zu verbessern.

Diese Aufgabe ist zunächst und im wesentlichen durch den Gegenstand des Anspruches 1 gelöst, wobei darauf abgestellt ist, dass der Stab mehrere am Stab hintereinander liegende Dosierkammern besitzt, die während einer Ausgabebetätigung nacheinander in die Entleerungsstellung treten und Stück für Stück von einem Luftüberdruck im Luftströmungskanal ausblasbar sind. Zufolge dieser Ausgestaltung ist eine aktive Entleerung jeder Dosierkammer erreicht. Die Substanzabgabe ähnelt der von mit Acrosolkartuschen versehenen Inhalatoren. Durch den Luftüberdruck wird die portionierte Substanz aus der Dosierkammer aktiv ausgestoßen, wobei durch Anordnung mehrerer hintereinander liegender Dosierkammern, die im Zuge einer Verlagerung des die Dosierkammern aufweisenden Stabes in dessen Längserstreckung nacheinander die Entleerungsstellung passieren, maschinengewehrartig mittels der Luft ausgeblasen werden. Eine Inhalation der Substanz ist aufgrund der aktiven Ausblasung der Dosierkammer bzw. der Dosierkammern auch dann zuverlässig durchführbar, wenn beispielsweise der Patient nicht in der Lage ist, einen genügend hohen Saugluftstrom zur Verlagerung und Leerung nur einer meist größeren Dosierkammer, wie aus dem Stand der Technik bekannt, zu erzeugen. Darüber hinaus ist durch die Luftdruckausblasung der Dosierkammern eine Feinstverteilung der ausgebrachten Substanz erreicht, was wiederum den Einsatz mikronisierter Pulversubstanzen ermöglicht, die auf direktem Wege lungenzugänglich eingestellt sind. So können weiter die Dosierkammern in einer solchen Größenordrung gewählt sein, dass je Kammer etwa 0,03 bis 0,2 mg Substanz aufnehmbar sind.

In einer bevorzugten Weiterbildung ist vorgesehen, dass der zur Inhalation der auszugebenden Substanz nötige Saugluftstrom den Entleerungsbereich des Luftströmungskanals, das heißt den durch den mit den Dosierkammern versehenen stabdurchsetzten Bereich nicht direkt um- bzw. durchströmt. Vielmehr wird der unterdruckbehaftete Saugluftstrom beim Einatmen nur im Bereich eines dem Mund oder der Nase zuzuordnenden Mischabschnittes aufgebracht, in welchen Mischabschnitt die abgeteilte Substanz aktiv über den Luftüberdruck eingeschossen wird.

Die Gegenstände der weiteren Ansprüche sind nachstehend in Bezug zu dem Gegenstand des Anspruchs 1 erläutert, können aber auch in ihrer unabhängigen Formulierung von Bedeutung sein.

So ist in einer Weiterbildung des Gegenstandes vorgesehen, dass der Stab als Flachstab ausgebildet ist, mit einer im Querschnitt gemessenen Länge, die einem Mehrfachen, so beispielsweise einem Zehnfachen bis Zwanzigfachen der quer hierzu gemessenen Breite entspricht. So ist ein Stab bevorzugt mit einer Stärke von etwa 0,3 bis 0,7 mm, bevorzugt 0,5 mm oder 0,3 mm vorgesehen. Der Flachstab kann aus einem Kunststoffmaterial, insbesondere aus einem Hartkunststoffmaterial bestehen. Bevorzugt ist eine Lösung, bei welcher der Flachstab aus einem metallischen Werkstoff gefertigt ist. Die in Längserstreckung des Flachstabes hintereinander liegenden Dosierkammern sind in einfachster Weise als im Grundriss kreisscheibenförmige Bohrungen vorgesehen mit jeweils einer Mittenachse, die senkrecht zu den Flachseiten des Flachstabes ausgerichtet sind.

Der Stab durchsetzt, insbesondere mit dem die Dosierkammern aufweisenden Abschnitt, die Substanzen-Vorratskammer unter Durchtauchen der in der Vorratskammer aufbewahrten pulverförmigen Substanz. Die Vorratskammerwand besteht hierbei bevorzugt aus einem elastischen Material, so beispielsweise aus einem gummiartigen Material, weiter beispielsweise aus einem thermoplastischen Elastomer. Bei Betätigung des Inhalators wird diese elastisch eingestellte Vorratskammerwand in Richtung des Stabes und somit in Richtung auf die Dosierkammern eingewölbt. Dies geschieht zunächst durch eine von radial außen auf die Vorratskammerwand einwirkende Überdruckbeaufschlagung, welcher Überdruck auch zur Entleerung der Dosierkammern in der Entleerungsstellung dient. Dieser Überdruck wird im Zuge der Inhalatorbetätigung aufgebaut, bevorzugt in Form eines Luftüberdrucks. Diesbezüglich ist weiter vorgesehen, dass sich die Vorratskammerwand mindestens im Dosierlochbereich bis zur Anlage am Stab einwölbt und so ein Eindrücken der in der Vorratskammer aufbewahrten Substanz in die Dosierkammern bewirkt. Im eingewölbten Zustand liegen auf jeder Stabflachseite, den jeweiligen Öffnungsquerschnitten der Dosierkammern zugewandt, Vorratskammerwandabschnitte an. In dieser Anlagestellung wirken die Vorratskammerwandabschnitte im Zuge einer Verlagerung des Stabes in die Entleerungsstellung abstreifend. Eine solche Abstreifung zur exakten Portionierung der auszugebenden Substanz erfolgt weiter bevorzugt noch vor Erreichen der Entleerungsstellung.

In einer bevorzugten Ausgestaltung des Erfindungsgegenstandes wird die Vorratskammerwand zusätzlich zu der beschriebenen Überdruckbeaufschlagung auch aktiv in Richtung auf den Stab eingewölbt. Hierzu sind Druckstücke vorgesehen, die von außen auf die Vorratskammerwand einwirken, zur bevorzugten Anlage von Wandabschnitten an den Stabflachseiten. Diese aktive Beaufschlagung der Kammerwand durch die Druckstücke unterstützt weiter die Befüllung der in dem zentral gehaltenen Stab ausgeformten Dosierkammern.

Die Verlagerung des die Dosierkammer aufweisenden Stabes in die Entleerungsstellung und die Druckbeaufschlagung der Vorratskammerwandung zur Befüllung der Dosierkammern erfolgen im Zuge jeweils einer Inhalatorbetätigung. Diesbezüglich ist ein Leerhub zwischen Inhalator-Betätigungstaste und dem Stab vorgesehen, der bewirkt, dass vor Mitnahme des Stabes die Einwölbung der Vorratskammerwand mittels der äußeren Druckstücke und gegebenenfalls luftdruckbeaufschlagt erfolgt. Entsprechend werden die Dosierkammern mittels der Druckstücke unter Zwischenschaitung der Vorratskammerwand bevorzugt noch in der Ruhestellung des Stabes befüllt. Erst nach dieser Befüllung und damit einhergehender Beendigung des Leerhubes wird der Stab durch Weiterverlagerung der Betätigungstaste mitgeschleppt, zur Verbringung der Dosierkammern in die Entleerungsstellung.

Die Druckstücke sind bevorzugt mit Backen ausgestattet, welche Stoßflächen besitzen, die in völlig eingeschwenkter bzw. angedrückter Stellung der Druckstücke parallel zur Breitseiten-Wandfläche des Stabes liegen, was zum Einen die korrekte Befüllung der Dosierkammern zur Folge hat. Zum Weiteren ist hierdurch eine Abstreifstellung erreicht. Die backenseitigen Stoßflächen sind entsprechend eine Ebene aufspannend flächig ausgebildet.

Die Betätigungstaste des Inhalators ist kopfseitig des Inhalatorgehäuses über dieses vorstehend ausgebildet und entgegen einer Rückstellfeder verlagerbar. So ist der gesamte Inhalator bevorzugt zwischen Daumen und beispielsweise Mittelfinger zu erfassen, wobei der Mittelfinger auf der Betätigungstaste ruht und der Daumen eine der Betätigungstaste gegenüberliegende Gehäusefläche abstützt. Die Inhalatorbetätigung erfolgt durch Druck auf die Betätigungstaste, wobei über Auflaufschrägen der Betätigungstaste die Druckstücke in Richtung des Stabes verschwenkt werden. Nach einem kurzen Leerhub schiebt die Betätigungstaste den Stab vor sich her unter Verlagerung der Dosierkammern aus der Befüllstellung in der Vorratskammer in die Entleerurigsstellung. Bei einer schwenkbaren Anordnung der Druckstücke wirken die Auflaufschrägen der Betätigungstaste entsprechend verschwenkend auf diese ein. Alternativ hierzu können die Druckstücke auch streng linear in radialer Richtung verlagerbar angeordnet sein, wobei mittels Keilwirkung diese Radialverlagerung zum Stab hin erreicht wird. Die Druckstücke sind insbesondere bei einer Schwenkausgestaltung derselben selbst rückstellend ausgebildet, so insbesondere durch eine entsprechende Federcharakteristik der die Druckstücke tragenden Arme. Entsprechend verfahren die Druckstücke nach Aufhebung der über die Auflaufschrägen einwirkenden Belastung wieder in ihre Ursprungsstellung zurück unter Freigabe der Vorratskammerwand, welch letztere sich aufgrund der elastischen Eigenschaften wieder in die Ursprungsstellung zurückstellt.

Bei aktiver Verlagerung der Betätigungstaste im Inhalatorgehäuse wird ein Luftüberdruck aufgebaut, der sich durch Verlagerung des Stabes an den Luftströmungskanal, konkret den sich in Strömungsrichtung an den Stab anschließenden Teil des Strömungskanals, zum Ausblasen der Substanz fortpflanzt. Entsprechend wird der Überdruck erst mit einer Verlagerung der Dosierkammer in den Luftströmungskanal unter Ausblasen der Substanz zumindest zum Teil abgebaut. Weiter entsprechend der Hintereinanderanordnung mehrerer Dosierkammern in dem Stab und des dementsprechend nacheinander geschalteten Anschließens der jeweiligen Dosierkammer an den Luftströmungskanal erfolgt der teilweise Abbau des Luftüberdruckes unter Ausschießen der Substanz aus der jeweiligen Dosierkammer stakkatoartig, was eine optimale Ausblasung der Substanz und wolkenartige Ausgabe an die Umgebung sowie an den durch Einatmen erzeugten Luftstrom bewirkt.

Der zum Ausblasen aufzubauende Luftüberdruck wird erreicht durch eine Kolben-/Gehäuseanordnung. Dies ist erreicht durch einen elastischen Kolbenring am einwärts liegenden Endbereich der topfförmigen Wand der Betätigungstaste, der mit einer Innenwand des Inhalatoraußengehäuses zusammenwirkt, welches Außengehäuse entsprechend einen Druckluftzylinder für den Kolbenring bildet. Die Betätigungstaste selbst formt innenwandig die Kolbenfläche aus. In bevorzugter Anordnung sind die Druckstücke und die Vorratskammer im Bereich des so geschaffenen Druckluftzylinders angeordnet, was die außenseitige Beaufschlagung der Vorratskammer und die Verlagerung der Druckstücke in die Dosierkammer-Befüllstellung unterstützt.

Der die Dosierkammern aufweisende, linear verlagerbare Stab besitzt Ventilcharakter. Der Querschnitt des Stabes verschließt den Luftauslass im Bereich des Luftströmungskanals, zufolge dessen sich abgewandt zur Ausgabeseite des Luftströmungskanals der Luftüberdruck im Zuge der Abwärtsverlagerung der Betätigungstaste aufbauen kann. Erst das Einfahren einer Dosierkammer des Stabes in den Luftströmungskanal bewirkt ein ventilartiges Öffnen und eine entsprechende Druckluftausblasung. Sind die Dosierkammern in Verfahrrichtung des Stabes entsprechend zueinander beabstandet, so erfolgt zwischen den ventilöffnungsartigen Ausblasphasen der Dosierkammern stets ein kurzfristiges Verschließen des Strömungsweges durch das Stabvollmaterial zwischen zwei Dosierkammer.

Zwischen der Luftauslass-Verschlussstelle, welche gebildet ist durch den Zusammenwirkungsbereich von Luftströmungskanal und den diesen quer durchsetzenden Stab, und dem die Vorratskammer aufnehmenden Gehäuseraum bzw. Druckluftzylinder ist ein Lufteinlassventil geschaltet, welches im Zuge einer federunterstützten Rückverlagerung der Betätigungstaste und entsprechender Vergrößerung des geschaffenen Zylinderraumes Luft nachströmen lässt, jedoch im Zuge einer aktiven Betätigung zur Ausgabe einer Inhalationssubstanz verschlossen ist.

Als besonders vorteilhaft erweist sich eine Weiterbildung des Erfindungsgegenstandes, bei welcher eine Einstellvorrichtung zur Bestimmung der Anzahl der in die Entleerungsstellung bringbaren Dosierkammern vorgesehen ist. So sind insbesondere bei einer solchen Ausgestaltung in dem Stab mehrere, so beispielsweise drei bis zehn oder mehr Dosierkammern in Verlagerungsrichtung betrachtet gleichmäßig zueinander beabstandet hintereinander liegend vorgesehen. Je nach Bedarf ist eine Dosierung wählbar, die die Verlagerung des Stabes in Längserstreckung begrenzt. Je nach Dosierbedarf werden entsprechend nur wenige oder mehr Dosierkammern in die Entleerüngsstellung, das heißt in den Bereich der Druckluft-Ausblasbeaufschlagung verbracht. Aufgrund der geringen Substanzmengen je Dosierkammer ist so eine sehr genaue, variable Dosierung erreichbar. So ist beispielsweise ein zugänglicher Einstellring vorgesehen, der mit mehreren Stufen versehen ist, zum Stoppen der Verschiebebewegung der Betätigungstaste. Entsprechend dem anschlagbegrerizten Verschiebeweg der Taste durchfahren eine bestimmte Anzahl von Dosierkammern die Entleerungsstellung. Zur Zusammenwirkung mit den einstellungsseitigen Stufen ist die Betätigungstaste mit Gegenmitteln versehen, so bevorzugt mit einem Stoppnocken.

Durch Querschnittsreduzierung des Luftströmungskanals, insbesondere des dem Ausblasende zugewandten Endabschnittes ist eine düsenartige Ausgestaltung erreichbar. So entspricht ein solch verringerter Querschnitt eines Luftströmungskanal-Endabschnittes etwa dem 0,5 bis 0,9-Fachen des ursprünglichen Querschnittes. In einer Weiterbildung des Erfindungsgegenstandes ist vorgesehen, dass der geradlinig quergerichtete Luftströmungskanal sich in einen querschnittskleineren, schräg aufwärts gerichteten Endkanalabschnitt fortsetzt, der innerhalb einer Nasentülle rückversetzt zu deren freien Ende endet. Zufolge dieser Ausgestaltung ist der vorgeschlagene Inhalator auch anwendbar zur nasalen Anwendung. Das zu inhalierende Medikament wird zufolge der aktiven Entleerung der Dosierkammern gezielt in den Nasenraum ausgeblasen. Das gleichzeitige Einatmen durch die Nase unterstützt vorteilhaft die Weiterverteilung des Medikaments. Durch die schräg aufwärts gerichtete Ausrichtung des Endkanalabschnitts und somit auch der diesen Endkanalabschnitt aufnehmenden Nasentülle ist der Inhalator bequem anwendbar, so weiter gegebenenfalls unter annähernd vertikaler Ausrichtung der Inhalatorachse während des Inhalationsvorganges.

Um dem Nutzer ein Mittel an die Hand zu geben, mittels welchem eine Füllstands- oder Abgabekontrolle ermöglicht ist, ist in einer Weiterbildung des Erfindungsgegenstandes ein bei Erreichen des Ausblasluftdruckes betätigtes Schrittschaltwerk vorgesehen. Ein solches Schrittschaltwerk zählt jede Einzelbetätigung des Inhalators, bei welcher eine Medikamentenausgabe erfolgt. So weist ein solches Schrittschaltwerk eine Skala auf, so beispielsweise einen Skalenring, auf welchem entweder die bereits durchgeführten Inhalationsvorgänge angezeigt werden oder alternativ abwärts zählend von einer Maximal-Inhalationszahl anzeigend die noch zur Verfügung stehenden Inhalationsschüsse. Das Schaltwerk wird in Abhängigkeit vom Erreichen des Ausblasluftdruckes betätigt, welcher Ausblasluftdruck unmittelbar vor Erreichen der gefüllten Dosierkammer in den Übergabebereich zum Luftströmungskanal aufgebaut ist. Demzufolge wird das Schrittschaltwerk unmittelbar vor Übergabe des Medikaments in den Luftströmungskanal und entsprechend unmittelbar vor einem aktiven Ausblasen des Medikaments geschaltet. Wird hingegen die Handhabe nur soweit betätigt, dass es nicht zu einem Ausschießen des Medikaments kommt, das heißt gegebenenfalls nur zu einer Befüllung der Dosierkammern und hiernach erfolgender Wiederrückverlagerung der Handhabe, erfolgt keine Schaltung des Schrittschaltwerks. Dies ist in einer beispielhaften Ausgestaltung durch einen auf das Schrittschaltwerk einwirkenden Zählfinger gelöst, dessen Spitze das Schrittschaltrad des Schrittschaltwerks etwa bei Beginn der Verlagerung des Stabes mit den gefüllten Dosierkammern in den Bereich des Luftströmungskanals erreicht. Der Zählfinger ist entsprechend mit Abstand zu dem Schrittschaltrad positioniert, welcher Abstand durch Handhabenbetätigung unter einhergehender Verlagerung des die Dosierkammern aufweisenden Stabes bei gleichzeitigem Aufbau des Ausblasluftdruckes überwunden wird.

Die Anzeige des Schrittschaltwerks ist in einer bevorzugten Ausgestaltung ein konzentrisch zur Inhalatorachse ausgerichteter Skalenring, welcher innenseitig zur schrittweisen Verlagerung in Umfangsdrehrichtung zahnkranzartig ausgeformt ist, zur Zusammenwirkung mit dem Schrittschaltrad. Letzteres ist in einer bevorzugten Ausgestaltung so ausgeformt, dass dieses den Skalenring des Schrittschaltwerkes schneckenradartig dreht. Es bedarf entsprechend keiner weiteren Zwischenübersetzungen zur Umsetzung der aus einer linearen Bewegung erfolgenden Schrittschaltfingerbetätigung des Schrittschaltrades in eine quer zur Achse gerichteten Kreisbewegung. Die Übertragung auf den Skalenring kann je nach Ausgestaltung insbesondere des Schrittschaltwerks unter- oder übersetzt sein.

Nachstehend ist die Erfindung anhand der beigefügten Zeichnung, welche lediglich mehrere Ausführungsbeispiele darstellt, näher erläutert. Es zeigt:
- Fig. 1: einen Vertikalschnitt durch einen Inhalator für pulverförmige Substanzen in einer ersten Ausführungsform, die unbelastete Grundstellung betreffend;
- Fig. 2: eine der Fig. 1 entsprechende Schnittdarstellung, jedoch nach Abnahme einer Mundstück-Schutzkappe, eine erste Zwischenstellung im Zuge einer Betätigung des Inhalators betreffend, in welcher Dosierkammern mit der Substanz befüllt werden;

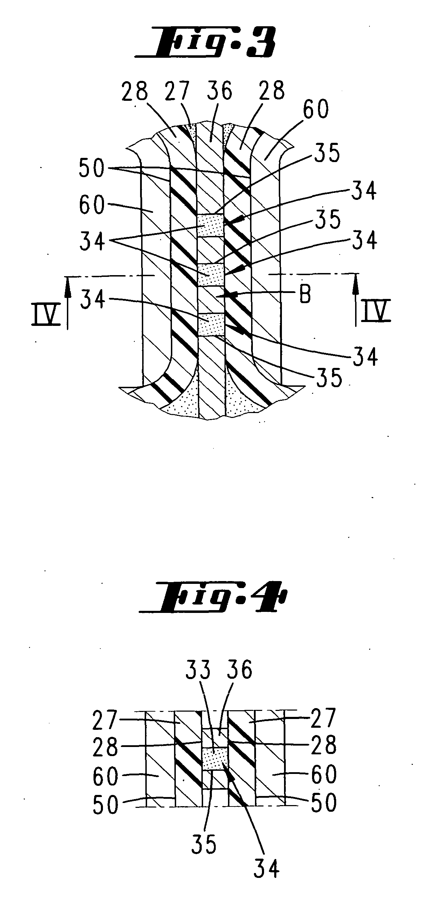

- Fig. 3: die Herausvergrößerung des Bereiches III in Fig. 2;
- Fig. 4: den Schnitt gemäß der Linie IV - IV in Fig. 3;
- Fig. 5: eine Folgedarstellung der Fig. 2, nach weiterer Betätigungsverlagerung und erfolgender Substanzabgabe;
- Fig. 6: den herausvergrößerten Bereich VI in Fig. 5;

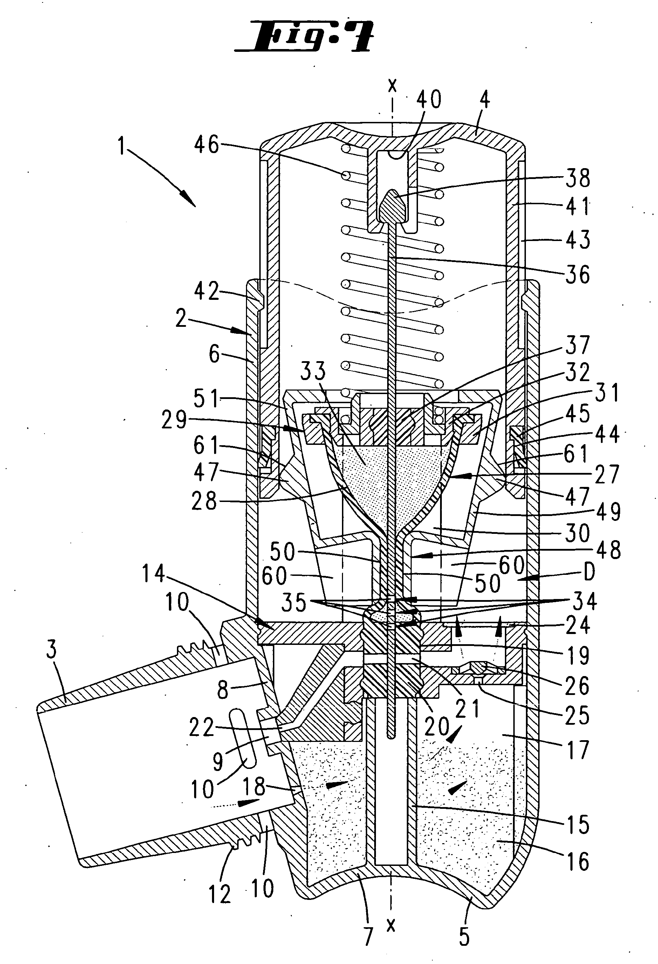

- Fig. 7: eine Folgedarstellung zu der Fig. 5, eine Zwischenstellung im Zuge der Rückverlagerung betreffend;

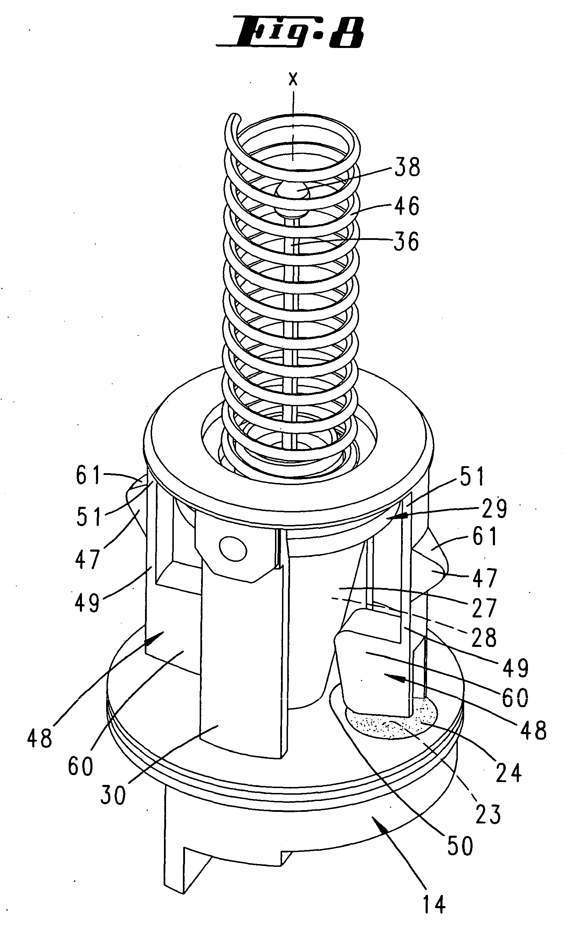

- Fig. 8: eine perspektivische Einzeldarstellung eines Halters mit an diesem angebrachter Vorratskammer und Druckstücken sowie einen Dosierkammern aufweisenden Stab und einer Rückstell-Wendelfeder;

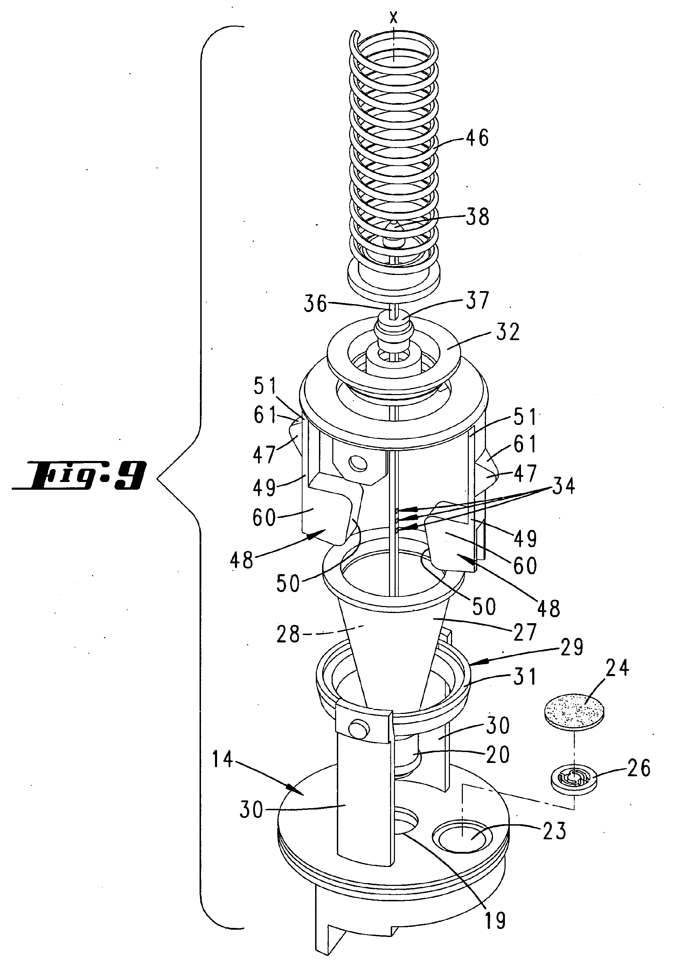

- Fig. 9: eine an die Fig. 8 angelehnte Darstellung, jedoch in Explosionsdarstellung;
- Fig. 10: in perspektivischer Darstellung den Inhalator in einer zweiten Ausführungsform;
- Fig. 11: den Inhalator gemäß Fig. 10 in einer Teil-Explosionsdarstellung.;
- Fig. 12: eine der Figur 1 entsprechende Vertikalschnittdarstellung, den Inhalator in einer weiteren Ausführungsform zur nasalen Inhalation betreffend;
- Fig. 13: den Inhalator in Vertikalschnittdarstellung in einer weiteren Ausführungsform mit einem Schrittschaltwerk, die Grundstellung betreffend;
- Fig. 14: den Bereich XIV in Fig. 13 in vergrößerter Darstellung;
- Fig. 15: eine im wesentlichen der Fig. 14 entsprechende Darstellung, jedoch in einer Betätigungsstellung des Inhalators, in welcher das Schrittschaltwerk durch einen Zählfinger betätigt ist;
- Fig. 16: eine perspektivische Teilschnittdarstellung gemäß Fig. 15;
- Fig. 17: den Schnitt gemäß der Linie XVII - XVII in Fig. 15;
- Fig. 18: das Schrittschaltrad des Schrittschaltwerks in einer Einzel-Seitenansichtsdarstellung und
- Fig. 19: das Schrittschaltrad in perspektivischer Einzeldarstellung.

Dargestellt und beschrieben ist ein Inhalator 1, welcher als bequem mitführbares Taschengerät realisiert ist, mit einem zylindrischen Gehäuse 2, von welchem ein annähernd radial abragendes Mundstück 3 ausgeht. Die allgemeine Form des Inhalators 1 entspricht in wesentlichen Zügen derer der mit Acrosolkartuschen versehenen Inhalatoren. Entsprechend ist auch die Handlungsweise des Inhalators 1 übertragen und für den Benutzer sofort erkennbar.

So besitzt der Inhalator 1 eine die allgemeine Gehäuseachse x aufnehmende, quer zu dieser Achse x ausgerichtete und über das Gehäuse 2 vorstehende Betätigungstaste 4, der eine gehäusefußseitige Gegenfläche 5 gegenübersteht. Durch eine Verlagerung der Betätigungstaste 4 in Richtung auf die unterseitige Gegenfläche 5 entlang der Achse x wird eine Substanzausgabe erreicht.

Das Gehäuse 2 ist in Form eines hohlzylindrischen Körpers gebildet, mit im dargestellten Ausführungsbeispiel kreisrundem Grundriss. Auch andere von dieser kreisrunden Grundrissform abweichende Formen sind denkbar, so weiter beispielsweise ellipsenartige oder auch mehreckige.

Das kreiszylinderförmige Inhalator-Außengehäuse 6 ist fußseitig geschlossen durch einen Inhalatorboden 7, der die Gegenfläche 5 zur Betätigung des Inhalators 1 bildet. Zur diesem Boden 7 gegenüberliegenden Seite hin ist das Gehäuse 2 offen gestaltet.

Im fußseitigen Bereich des Gehäuses 2 wächst aus diesem etwa in radialer Ausrichtung, so weiter konkret im dargestellten Ausführungsbeispiel unter Einschluss eines spitzen Winkels von etwa 75 bis 80° zur Inhalatorachse x, das Mundstück 3 aus, welches im wesentlichen als Hohlzylinderkörper ausgebildet ist, mit einer mit Bezug zu der Ausrichtung des Mundstückes 3 nach axial außen weisenden Mündung. Ein im Übergangsbereich vom Gehäuse 2 in das Mundstück 3 angeordneter Mundstückboden 8 besitzt eine zentrale Öffnung 9. Mit axialem Abstand zu dieser zentralen Bodenöffnung 9 sind in der Mundstückwändung entlang einer Umfangslinie gleichmäßig verteilt angeordnete Lufteinströmöffnungen 10 ausgeformt, zum Luftströmungsanschluss des der Bodenöffnung 9 zugeordneten Mundstückinnenraumes an die Umgebung.

Das Mundstück 3 ist bei Nichtgebrauch des Inhalators 1 durch eine Schraubkappe 11 überfangen. Hierzu ist das Mundstück 3 mantelaußenwandig mit einem Außengewinde 12 versehen, welches mit einem Innengewinde 13 der Kappe 11 zusammenwirkt. Der das Innengewinde 13 aufweisende Mantelabschnitt der Schraubkappe 11 überfängt in der Verschlussstellung die Lufteinströmöffnungen 10 des Mundstückes 3, wobei weiter die Stirnringfläche dieses Mantelabschnitts der Schraubkappe 11 anschlagbegrenzt gegen einen Gehäuseabschnitt tritt.

Das Gehäuse 2 ist quer zur Gehäuseachse x unterteilt durch einen an der Gehäuseinnenwand auf Höhe des Überganges vom Gehäuse zum Mundstück 3 befestigten Träger 14. Es ergibt sich entsprechend ein mit Bezug zu dem Träger 14 oberer Gehäuseabschnitt und ein unterer, der Gegenfläche 5 zugeordneter Abschnitt, welch letzterer zentral durchsetzt ist von einem sich in Achsrichtung erstreckenden Stützrohr 15, auf welchem der Träger 14 aufsitzt. In dem hierdurch geschaffenen Ringraum im unteren Gehäuseabschnitt ist ein feuchteabsorbierendes Material 16 aufgenommen. Des weiteren steht dieser Ringraum 17 über eine Nachströmöffnung 18, welche im Bereich des Mundstückbodens 8 ausgebildet ist, mit dem Zylinderinnenraum des Mundstückes 3 und über die Lufteinströmöffnungen 10 mit der Umgebung in Verbindung.

Der scheibenförmige, massive Träger 14 besitzt eine zentrale Aufnahme 19, in die ein aus einem thermoplastischen Material bestehendes Dichtelement 20 eingesetzt ist. Dieses Dichtelement 20 sitzt stopfenartig in der Aufnahme 19, wobei weiter der Träger 14 über dieses Dichtelement 20 sich auf dem Rohr 15 abstützt. Das Dichtelement 20 ist mit einem im wesentlichen geradlinig quer zur Achse x ausgerichteten Luftströmungskanal 21 versehen, der beidseitig, den Träger 14 jeweils durchsetzend, weitergeführt ist. So erstreckt sich der Luftströmungskanal 21 einerseits des Dichtelements 20 den Träger 14 durchsetzend bis hin zu der zentralen Öffnung 9 des Mundstückbodens 8, zur Bildung eines Luftauslasses 22. In entgegengesetzter Richtung mit Bezug zum Dichtelement 20 setzt sich der Luftströmungskanal 21 unter Erweiterung seines Querschnittes hin zum durch den Träger 14 abgetrennten oberen Gehäuseabschnitt fort. Die entsprechende Kanalmündung 23 ist auf der dem oberen Gehäuseabschnitt zugewandten Breitfläche des Trägers 14 ausgebildet, wobei weiter diese Kanalmündung 23 von einem Filterelement 24 überdeckt ist.

Der Luftströmungskanal 21 ist somit unterteilt in einen mundstückseitigen Kanalabschnitt und einen gehäuseseitigen Abschnitt. In letzterem ist die filterüberdeckte Kanalmündung 23 ausgeformt. Weiter ist in diesem Abschnitt eine der Kanalmündung 23 gegenüberliegende Nachströmöffnung 25 angeordnet, die eine Verbindung zwischen dem gehäuseseitigen Abschnitt des Luftströmungskanals 21 und dem unterseitig des Trägers 14 ausgeformten Ringraum 17 bildet. Diese Nachströmöffnung 25 ist überfangen von einem Lufteinlassventil 26, welches so geschaltet ist, dass die Nachströmöffnung 25 nur bei einer Luftströmung vom Ringraum 17 durch den Luftströmungskanal 21 in Richtung auf den oberen Gehäuseabschnitt geöffnet wird. In entgegengesetzter Luftströmungsrichtung verschließt das Ventil 26 diese Nachströmöffnung 25.

Der Luftströmungskanal 21 insbesondere im Bereich des Dichtelements 20 und des dem Mundstück 3 zugewandten Abschnitts ist wesentlich kleiner bemessen als der freie Querschnitt des Mundstückes 3. So entspricht der freie Innenraumdurchmesser des Mundstückes 3 etwa dem Zehn- bis Dreißigfachen des Durchmessers des Luftströmungskanals 21, welch letzterer insbesondere ausgehend von dem Dichtelement 20 in Richtung auf die mundstückseitige Öffnung 9 im Bereich eines schräg abwärts verlaufenden Abschnitts sich hin verjüngend ausgebildet ist, zur Ausformung eines düsenartigen Kanals.

Das Dichtelement 20 geht einstückig, materialeinheitlich über in eine dem oberen Gehäuseabschnitt zugewandte, trichterförmige Vorratskammer 27, mit sich nach oben, das heißt in Richtung auf die stirnseitige Gehäuseöffnung hin sich erweiterndem Querschnitt. Die Vorratskammerwand 28 besteht entsprechend auch aus einem thermoplastischen. Elastomer oder einem anderen gummiartigen Werkstoff.

Das durchmessererweiterte freie Ende der Vorratskammer 27 ist erfasst von einem quer zur Inhalatorachse x ausgerichteten Halter 29, der über seitlich der Vorratskammer 27 sich erstreckende, gegenüberliegend zueinander angeordnete Stützen 30 an dem das untere Ende der Vorratskammer 27 fassenden Träger 14 befestigt ist. Entsprechend ist der axiale Abstand der Vorratskammerenden zueinander fixiert.

Das obere freie Ende der Vorratskammer 27 ist dichtend in dem Halter 29 gefasst, so in dem dargestellten Ausführungsbeispiel eingeklemmt zwischen einem radial äußeren Haltering 31 und einem radial inneren, stopfenartigen HaIteabschnitt 32.

In der Vorratskammer 27 ist eine mikronisierte pulverförmige Substanz 33 aufbewahrt, welche mittels des vorgeschlagenen Geräts in portionierter Ausgabe inhaliert werden soll.

Zur portionierten Ausgabe der Substanz 33 sind Dosierkammern 34 vorgesehen, so in der dargestellten ersten Ausführungsform deren drei. Die Größe einer jeden Dosierkammer 34 definiert die jeweils auszutragende Substanzmenge.

Die Dosierkammern 34 sind als Querbohrungen 35 eines als Flachstab gebildeten, zentral sich entlang der Achse x erstreckenden Stabes 36 ausgebildet. Die Querbohrungen 35 durchsetzen hierbei die Breitseitenwandflächen des Flachstabes 36, wobei weiter dieser im Querschnitt ein Breiten-/Längenverhältnis von 1 : 5 bis 1 : 20 aufweist. In dem dargestellten Ausführungsbeispiel ist eine Flachstabstärke von etwa 0,5 mm gewählt, bei einer quer hierzu gemessenen Länge von etwa 3 bis 3,5 mm. Die Querbohrungen 35 sind in ihrem Durchmesser so gewählt, dass je hierdurch gebildete Dosierkammer 34 eine Substanzmenge von 0,05 mg bis 0,1 mg aufnimmt.

Der Stab 36 mit den Dosierkammern 34 durchsetzt die Vorratskammer 27 zentral in Erstreckungsrichtung der Achse x. Fußseitig der Vorratskammer 27 durchsetzt der Stab 36 weiter das Dichtelement 20 unter Querung des in diesem ausgeformten Luftströmungskanals 21, zufolge dieser Ausgestaltung mittels des Stabes 36 ein Verschluss des Luftströmungskanals 21 zunächst erreicht ist.

In hierzu entgegengesetzter Richtung erstreckt sich der Stab 36 über die Vorratskammer 27 hinaus, unter Durchsetzung des die Vorratskammer 27 festlegenden Halters 29. Im Durchsetzungsbereich des Halters 29 ist ein weiteres Dichtelement 37 angeordnet, welches wie auch das Dichtelement 20 im fußseitigen Bereich der Vorratskammer 27 abstreifend auf die Oberflächen des Stabes 36 einwirkt, unter gleichzeitiger Abdichtung der Durchsetzungszonen.

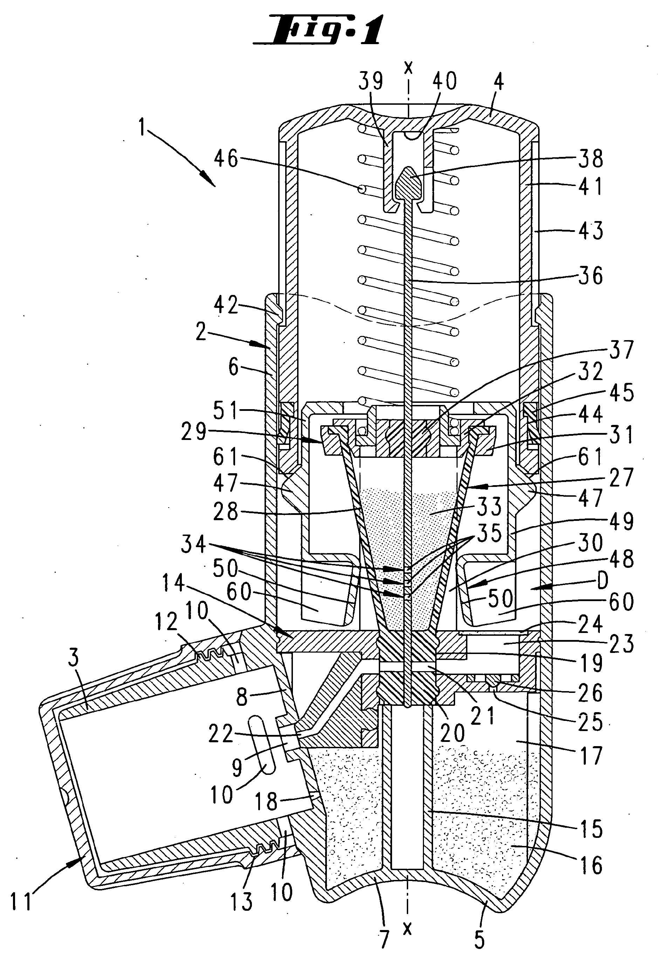

Die in Längserstreckung des Stabes 36 gleichmäßig zueinander beabstandeten, hintereinander angeordneten Dosierkammern 34 sind in einer Grundstellung des Inhalators 1 gemäß der Darstellung in Figur 1 im unteren Drittel der Vorratskammer 27 positioniert, umschlossen von der bevorrateten Substanz 33. Der Abstand der Dosierkammern 34 zueinander entspricht im wesentlichen etwa dem Durchmesser einer eine Dosierkammer 34 ausformenden Querbohrung 35.

Das nach oben über die Vorratskammer 27 hinaus ragende freie Ende des Stabes 36 ist mit einem pilzkopfartigen Mitnehmer 38 versehen. Dieser ist erfasst von unterseitig der Betätigungstaste 4 angeformten Schlepparmen 39, die eine Länge aufweisen, die etwa der zweifachen Länge des Mitnehmers 38 in Erstreckungsrichtung der Achse x entspricht. So ist ein Leerhub geschaffen zwischen der der Betätigungstaste 4 zugewandten Spitze des Mitnehmers 38 und der zu dieser korrespondierenden unterseitigen Mitnahmefläche 40 der Betätigungstaste 4.

Die im wesentlichen sich quer zur Inhalatorachse x erstreckende Betätigungstaste 4 geht über in einen konzentrisch zur Achse x ausgeformten zylinderartigen Abschnitt mit einer topfförmigen Wandung 41, die mit ihrer Öffnung nach unten weisend in das Gehäuse 2 eintaucht. Der Außendurchmesser der Wandung 41 ist entsprechend an den Innendurchmesser des Gehäuse-Zylinderabschnittes 6 angepasst. Die Betätigungstaste 4 ist mit ihrer Wandung 41 unter Führung durch den Zylinderabschnitt 6 in das Gehäuse 2 einschiebbar, dies bei Anschlagbegrenzung in den jeweiligen Endstellungen.

Zur Realisierung dieser Anschlagbegrenzung weist der Gehäuse-Zylinderabschnitt 6 im Bereich seines freien Randabschnittes zwei diametral gegenüberliegende, radial nach innen weisende Führungsnasen 42 auf, die in achsparallele Nuten 43 im Bereich des Außenmantels der Wandung 41 eingreifen. Hierdurch ist auch eine Verdrehsicherung zwischen Gehäuse 2 und Betätigungstaste 4 erreicht.

Im Bereich des in das Gehäuse 23 einragenden freien Endes der Betätigungstastenwandung 41 ist mantelaußenwandig eine Ringnut 44 vorgesehen, zur Aufnahme eines aus einem Elastomermaterial bestehenden Kolbenringes 45, der zur Abdichtung gegen die Innenwandung des Gehäuse-Zylinderabschnittes 6 tritt.

Die Betätigungstastengrundstellung gemäß der Darstellung in Figur 1 ist unterstützt durch eine unterseitig auf die Betätigungstaste 4 einwirkende Rückstell-Wendelfeder 46, die den Stab 36 sowie die Schlepparme 39 der Betätigungstaste 4 umgebend anderendig sich auf dem den kopfseitigen Abschluss der Vorratskammer 27 bildenden Halter 29 abstützt. Diese Grundstellung ist durch Anschlagen der Führungsnasen 42 am unteren Ende der betätigungstastenseitigen Nuten 43 definiert, wobei weiter in dieser Grundstellung der stabseitige Mitnehmer 38 einen maximalen Abstand zur Mitnahmefläche 40 der Betätigungstaste 4 einnimmt.

In den Verlagerungsweg der Betätigungstasten-Wandung 41 treten keilförmige Ausrückvorsprünge 47 mit nach oben weisenden Auflaufschrägen 61 zweier diametral sich gegenüberliegender, im fußseitigen freien Endbereich nach radial innen ragende Druckstücke 48 tragender Arme 49. Diese mit den Druckstücken 48 versehenen Arme 49 sind mit Bezug auf einen Grundriss um 90° versetzt zu den den Halter 29 tragenden Stützen 30 positioniert; weiter an diesen über einen im Grundriss kreisringförmigen Träger an dem Halter 2 befestigt. Die Arme 49 sowie die nach radial innen weisenden Stoßflächen 50 der Backen 60 formenden Druckstücke 48 erstrecken sich in einem Grundriss bzw. in einem Querschnitt durch den Inhalator 1 parallel beabstandet zu einer Breitseitenfläche des Stabes 36. Entsprechend sind die Stoßflächen 50 den Breitseitenflächen des Stabes 36 zugewandt positioniert, wobei weiter die Stoßflächen 50 jeweils eben ausgebildet sind.

Insbesondere die Arme 49, weiter insbesondere die Anlenkungsbereiche 51 an den kreisringförmigen Träger sind hinsichtlich der Materialwahl und/oder hinsichtlich der Materialstärke so gewählt, dass um die Anlenkungsbereiche 51 ein radiales Einschwenken in Richtung auf die Achse x zugelassen ist. Die Federeigenschaften des gewählten Kunststoffmaterials werden genutzt zur selbsttätigen Rückstellung der Arme 49 in die Ursprungsstellung.

Die in axialer Richtung gemessene Länge der Arme 49 ist so gewählt, dass die endseitig vorgesehenen Druckstücke 48 sich etwa auf Höhe des unteren Drittels der Vorratskammer 27 erstrecken.

Die Funktionsweise des Inhalators 1 ist wie folgt:

Durch Druck (dargestellt durch den Pfeil P) auf die Betätigungstaste 4 wird diese entlang der Achse x schiebeverlagert in das Gehäuse 2 abgesenkt. Gehäuse 2 und bedingt durch die Abdichtung über den Kolbenring 45 die topfartige Betätigungstaste 4 bilden einen Druckluftzylinder D, in welchem im Zuge der Absenkung der Betätigungstaste 4 ein Luftüberdruck aufgebaut wird. Die innenliegende Unterseite der Betätigungstaste 4 formt hierbei die Kolbenfläche.

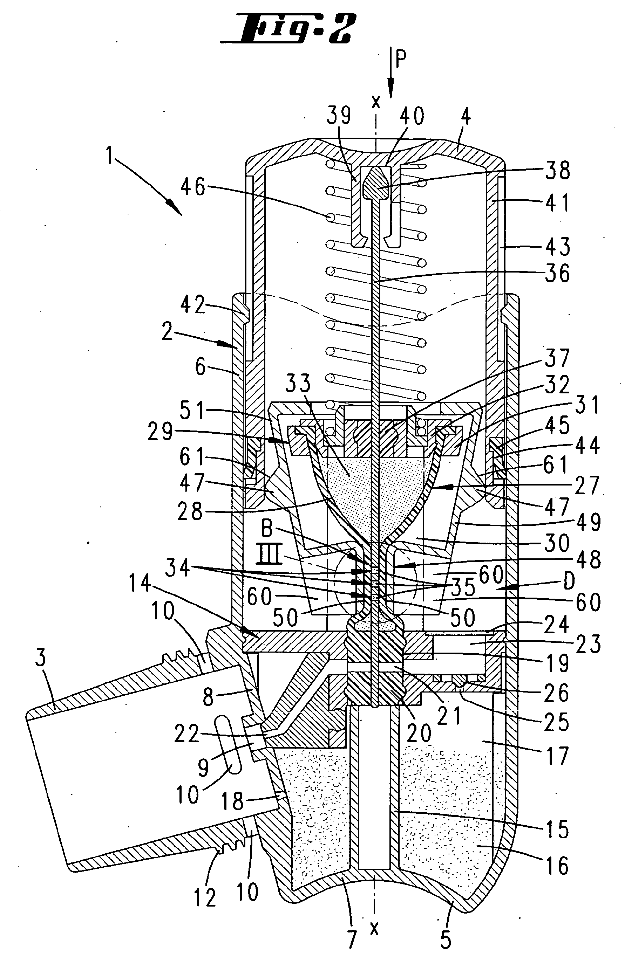

Zudem werden im Zuge der Abwärtsbewegung der Betätigungstaste 4 über die mit einer entsprechenden Fase versehenen Stirnrandkante der Wandung 41 die Ausrückvorsprünge 47 beaufschlagt, was unter weiterer Absenkung der Taste 4 zu einem Einschwenken der Arme 49 um die Anlenkbereiche 51 führt. Infolge dessen schwenken die Drückstücke 48 um einen Radius zu den Anlenkbereichen 51 nach radial innen unter Einwölbung der Vorratskammerwand 28 bis in die Befüllungsstellung B gemäß der Darstellung in Figur 2, in welcher die Stoßflächen 50 in Parallelausrichtung zueinander und zu den Breitseitenflächen des Stabes 36 gelangen, in welcher Stellung unter Zwischenlage der jeweiligen Vorratskammerwandabschnitte Substanzportionen in die Dosierkammern 34 eingedrückt sind. Die in der Grundstellung des Inhalators 1 beidseitig der Dosierkammeröffnungen vorliegende Substanz wird mittels der Vorratskammerwand 28 und der auf diese einwirkenden Druckstücke 48 in die Querbohrungen 35 gedrängt, wonach insbesondere bei einer mikronisierten Pulversubstanz eine Selbsthaltung in den Dosierkammern 34 gegeben ist.

Die Einwölbung der Vorratskammerwand 28 zum Eindrücken der Substanz in die Dosierkammern 34 wird unterstützt durch den sich im Zuge dieses Vorgangs aufbauenden Luftüberdruckes in dem Druckluftzylinder D.

Bis zur vollständigen Befüllung der Dosierkammern 34 verharrt der Stab 36 in seiner Grundstellung, dies bedingt durch den im Bereich der Schlepparme 39 vorgesehenen Leerhub. Erst nach dieser Dosierkammerbefüllung tritt die unterseitig der Taste ausgeformte Mitnahmefläche 40 gegen den stabendseitigen Mitnehmer 38, zur Mitschleppung des Stabes 36 bei weiterer Abwärtsverlagerung der Betätigungstaste 4.

Im Zuge dieser Stababwärtsverlagerung nach Dosierkammerbefüllung treten die befüllten Dosierkammern 34 nacheinander in Überdeckung zu dem Luftströmungskanal 21, der bis dahin durch den geschlossenen, massiven Endabschnitt des Stabes 36 schieberartig verschlossen ist, was den Druckaufbau ermöglicht. Erreicht eine Dosierkammer 34 den Luftströmungskanal 21 (Entleerungsstellung E), so wird das so geschaffene Ventil kurzzeitig geöffnet. Die die Dosierkammer 34 ausformende Querbohrung 35 wird Teil des Luftströmungskanals 21. Der aufgebaute Luftüberdruck bewirkt ein schlagartiges Ausblasen der portionierten Substanz aus der Dosierkammer zum Eindüsen dieser Portion über den Luftauslass 22 in das Mundstück 3, welch letzteres bei einer Betätigung des Inhalators 1 von Lippen umschlossen ist derart, dass die Lufteinströmöffnungen 10 nicht überdeckt werden. Durch Einatmen wird über diese Lufteinströmöffnungen 10 Umgebungsluft angesaugt, die mit der eingedüsten portionierten Substanzwolke angereichert wird.

Entsprechend der in dem Ausführungsbeispiel dargestellten Anordnung von drei hintereinander vorgesehenen Dosierkammer 34 erfolgt in Abhängigkeit von der Geschwindigkeit der Abwärtsverlagerung der Betätigungstaste 4 ein sehr schnelles, jeweils kurzzeitiges druckluftunterstütztes Ausstoßen der Substanzportionen.

Das in Axialrichtung nach unten ausweichende freie Ende des Stabes 36 tritt im Zuge der Abwärtsverlagerung in den Innenraum des Stützrohres 15 ein.

Die in Abwärtsverlagerungsrichtung betrachtete Endstellung der Betätigungstaste 4 ist gleichfalls anschlagbegrenzt. Dies durch Anschlagen der Führungsnasen 42 an den oberen Randbereich der mit diesen zusammenwirkenden Nuten 43 und/oder durch Treten der freien Ringstirnfläche der Betätigungstasten-Wandung 41 gegen die Oberfläche des Trägers 14.

Durch Aufhebung der Druckbeaufschlagung der Betätigungstaste 4 stellt sich diese samt dem mitgeschleppten Stab 36 bedingt durch die aufgebaute Federkraft selbsttätig in die Grundstellung zurück. Dies unter Freigabe der die Druckstücke 48 aufweisenden Arme 49, die entsprechend auch aufgrund der Federcharakteristik des gewählten Materials in ihre Ursprungsstellung zurück verschwenken.

Im Zuge der Rückverlagerung der Betätigungstaste 4 und der damit einhergehenden Vergrößerung des Volumens des Druckluftzylinders D wird Luft nachgesaugt. Dies über die Nachströmöffnungen 18 und 25 unter Durchströmen des feuchteabsorbierenden Materials 16 bei entsprechender Öffnung des Lufteinlassventils 26.

Bedingt durch die trichterförmige Ausgestaltung der Vorratskammer 7 rutscht Substanzmaterial nach Aufheben der äußeren Belastung auf die Vorratskammerwand 28 mittels der Druckstücke 48 selbsttätig nach, wobei weiter durch die Beeinflussung der Vorratskammerwand 28 durch Einwölbung ein solches Substanznachrücken durch Walken unterstützt wird.

Mit Ausnahme der Dichteigenschaften aufweisenden Elemente und der Vorratskammer 27, gegebenenfalls auch mit Ausnahme des eine Federcharakteristik aufweisenden Bauteils mit Armen 49 und Druckstücken 48 ist der Inhalator 1, insbesondere das Gehäuse und die Betätigungstaste 4 mit der Wandung 41 sowie der Halter 29 mit dem Träger 14, aus einem Kunststoffmaterial, weiter insbesondere aus einem Hartkunststoffmaterial, gefertigt. Auch der Stab 36 kann aus einem solchen Hartkunststoffmaterial bestehen. Bevorzugt wird diesbezüglich jedoch ein Stab 36 aus einem Metallwerkstoff.

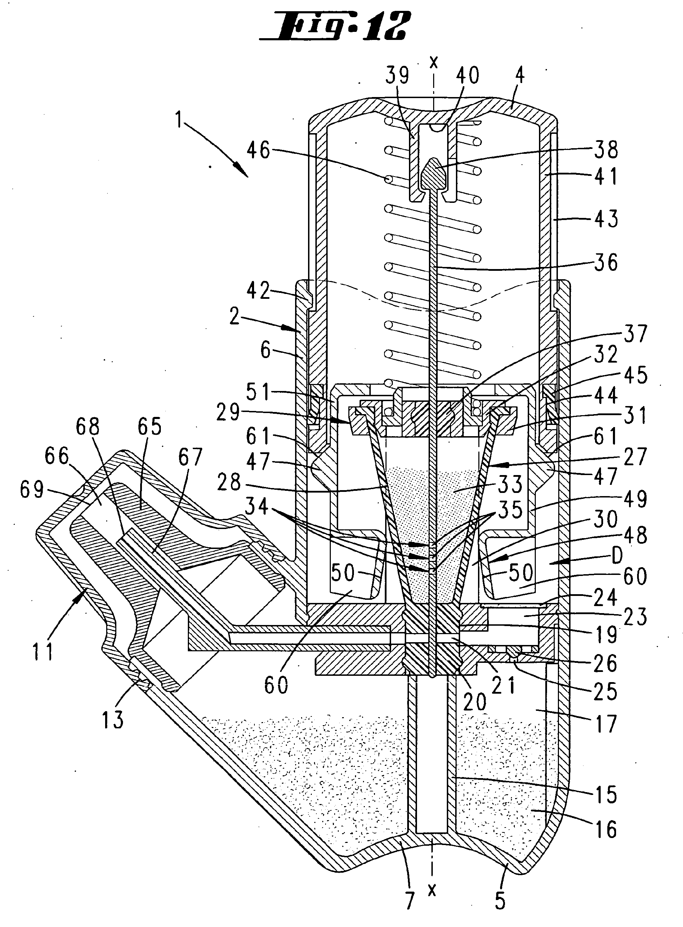

Darüber hinaus ist der zur Oralbehandlung ausgeformte Inhalator 1 auch in einer Ausführung zur nasalen Inhalation denkbar. So wie bspw. in Figur 12 dargestellt.

Gemäß der Darstellung in Figur 12 ist bei einer Ausführungsform zur nasalen Inhalation der Ausgabe- bzw. Ausstoßbereich mit Bezug auf die Vertikalachse x nach schräg oben gerichtet, so unter Einschluss eines Winkels zur Längsachse x von etwa 45°. Das Gehäuse 2 ist hierzu mit einer Nasentülle 65 versehen, welche bei Nichtbenutzung des Inhalators 1 entsprechend der zuvor beschriebenen Ausführungsform von einer Schraubkappe 11 überdeckt ist.

Die Nasentülle 65 ist in ihrer Längserstreckung und in ihrem Außendurchmesser angepasst zur Einführung in eine Nasenöffnung. Zentral in Längserstreckung der Nasentülle 65 ist diese durchsetzt von einem Ausgabekanal 66. In diesen mündet ein Endkanalabschnitt 67 des Luftströmungskanals 21, dessen freies Ende 68 rückversetzt, entsprechend mit Bezug auf die Nasentüllen-Körperachse axial rückverlagert zu dem freien Ende 69 der Nasentülle 65 platziert ist.

Entsprechend der Ausrichtung der Nasentülle 65 bzw. deren im spitzen Winkel zur Längsachse x ausgerichteten Körperachse verläuft auch der Endkanalabschnitt 67 ausgehend von dem quer gerichteten geradlinigen Luftströmungskanal 21 schräg aufwärts gerichtet, dies unter Verringerung des freien Querschnittes gegenüber dem Querschnitt des Luftströmungskanals 21 um etwa ein Drittel, was eine düsenartige Ausgestaltung des Endkanalabschnitts 67 zulässt.

Durch die mit Bezug auf die Gehäuseachse x schräg aufwärts gerichtete Anordnung von Endkanalabschnitt 67 und Nasentülle 65 ist eine Handhabung des Inhalators 1 zur nasalen Inhalation bei insgesamt etwa senkrechter Ausrichtung des Gehäuses 2 entlang der Gehäuseachse x erreichbar.

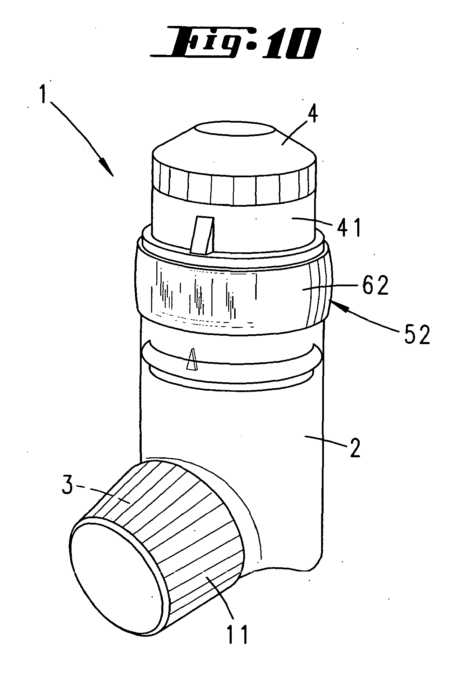

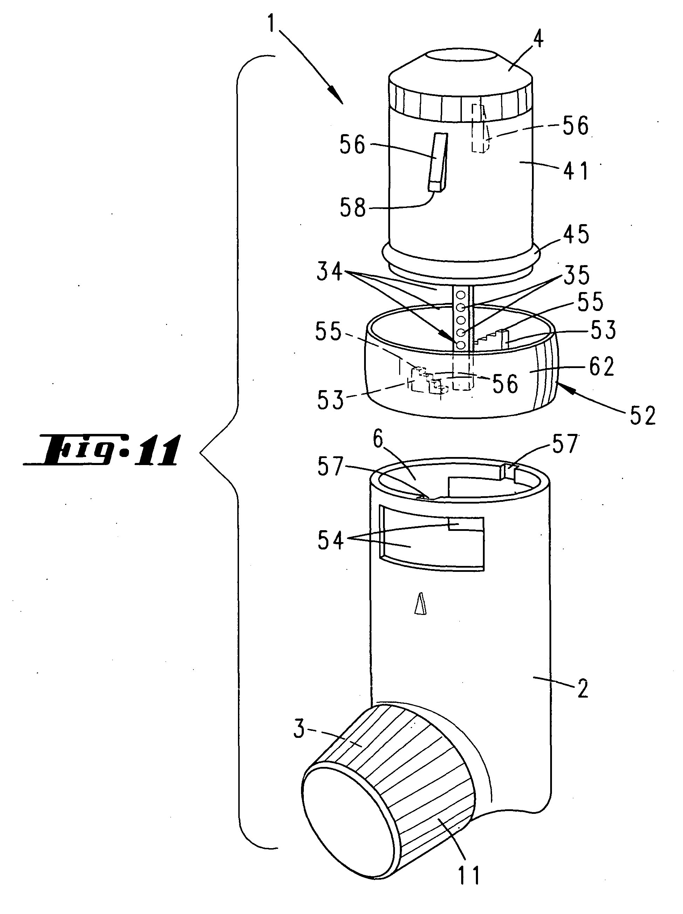

In den Figuren 10 und 11 ist eine zweite Ausführungsform dargestellt, betreffend einen Inhalator 1 mit einer Dosiereinstellung.

Es ist eine ringartige, das Gehäuse 2 im oberen, der Betätigungstaste 4 zugewandten Randbereich umfassende Einstellvorrichtung 52 vorgesehen. Deren Einstellring 62 ist koaxial zur Achse x um diese stufenweise drehbar, so beispielsweise über vier Drehraststufen zur Einstellung von fünf unterschiedlichen Dosierungen.

Mantelinnenseitig ist die ringförmige Einstellvorrichtung 52 mit diametral gegenüberliegend angeordneten Stufenkörpem 53 versehen, welche nach radial innen ragend Fenster 54 des Gehäuses 2 durchsetzend ausgebildet sind.

jeder Stufenkörper 53 ist mit stufenweise zueinander versetzten Anschlagflächen 55 versehen. Die Anzahl von Stufenflächen entspricht der Anzahl von Dosiereinstellmöglichkeiten. Diese Anschlagflächen 55 wirken zusammen mit entsprechend diametral gegenüberliegend an dem Außenmantel der Wandung 41 angeordneten Nocken 56. Diese sind in entsprechend positionierten Nuten 57 wandungsinnenseitig des Gehäuse-Zylinderabschnittes 6 geführt und gegen Verdrehen gegenüber dem Gehäuse 2 gesichert.

Eine nach unten in Einschiebrichtung der Betätigungstaste 4 weisende Nockenfläche 58 tritt bei Betätigung gegen die in den Verlagerungsweg des Nockens 56 eingedrehte Anschlagfläche 55 des Stufenkörpers 53, womit die Verschiebebewegung der Betätigungstaste 4 gestoppt ist.

Damit einhergehend ist auch die Verlagerung des in diesem Ausführungsbeispiel mit fünf hintereinander angeordneten Dosierkammern 34 versehenen Stabs 36 anschlagbegrenzt, zufolge dessen in Abhängigkeit von der Einstellung über die Stufenanschläge die Anzahl der in Entleerungsstellung E zu bringenden Dosierkammern 34 vorwählbar ist.

Entsprechend der Vorgabe über die Einstellvorrichtung 52 sind bei einer Betätigung des Inhalators 1 beispielsweise nur zwei Dosierkammern 34 oder nur die erste Dosierkammer 34 an den Luftströmungskanal 21 anschließbar und mittels Druckluftbeaufschlagung ausblasbar. In einer weiteren Einstellung können beispielsweise alle Dosierkammern 34, so wie dargestellt fünf Dosierkammern 34, nacheinander dem Luftströmungskanal 21 zugeführt werden und deren Substanzportionen salvenartig ausgeschossen werden.

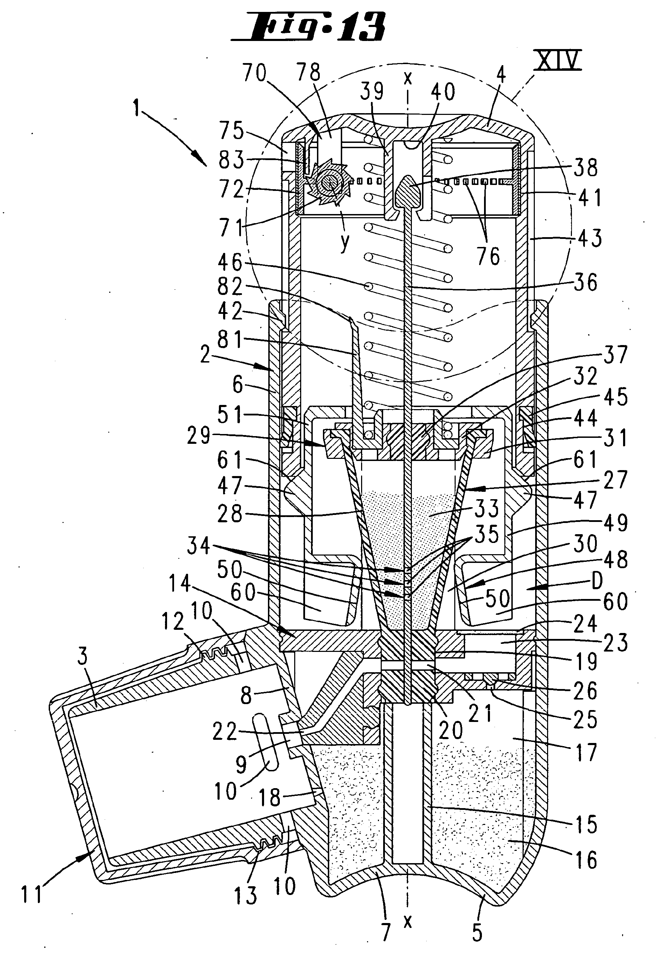

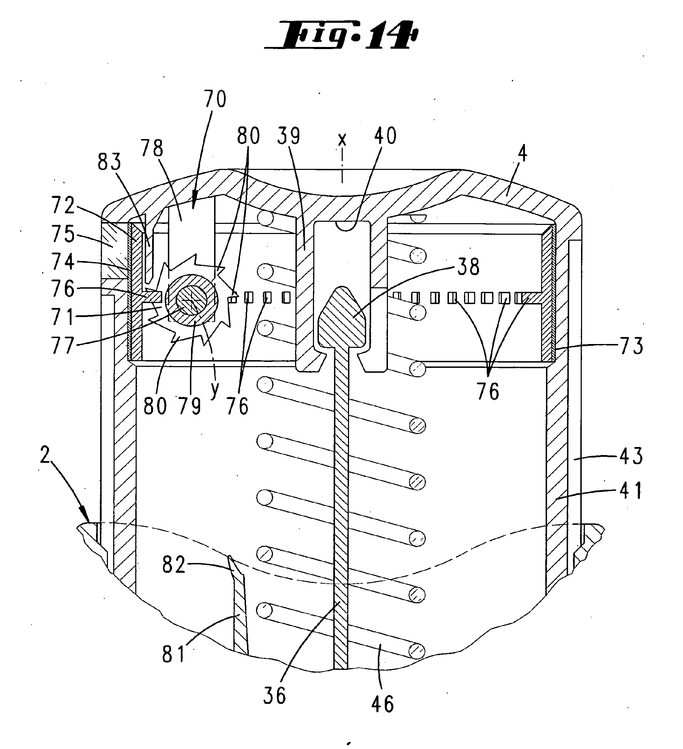

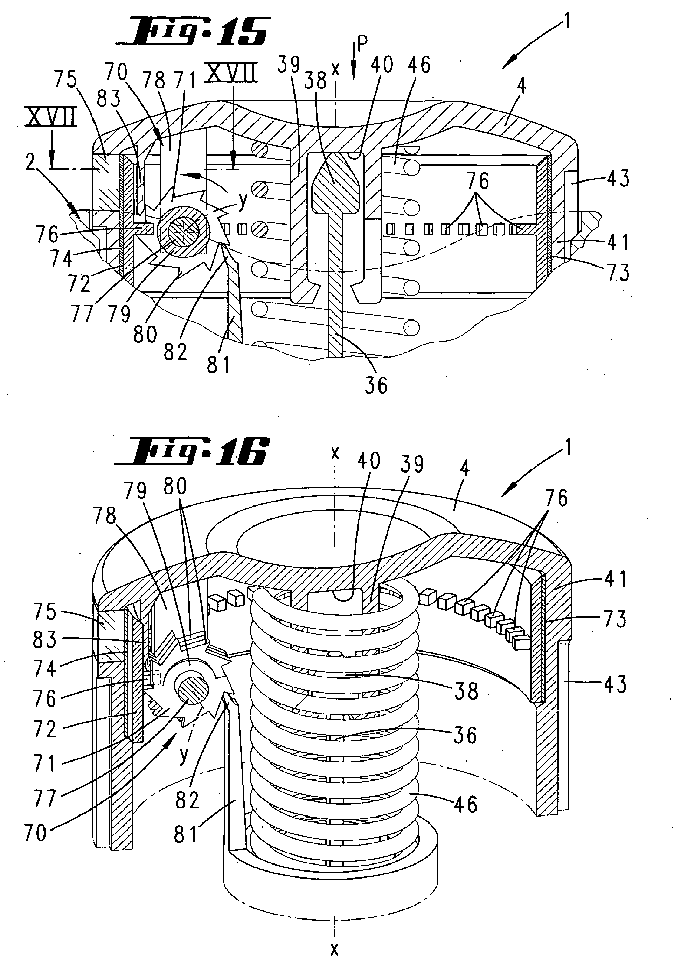

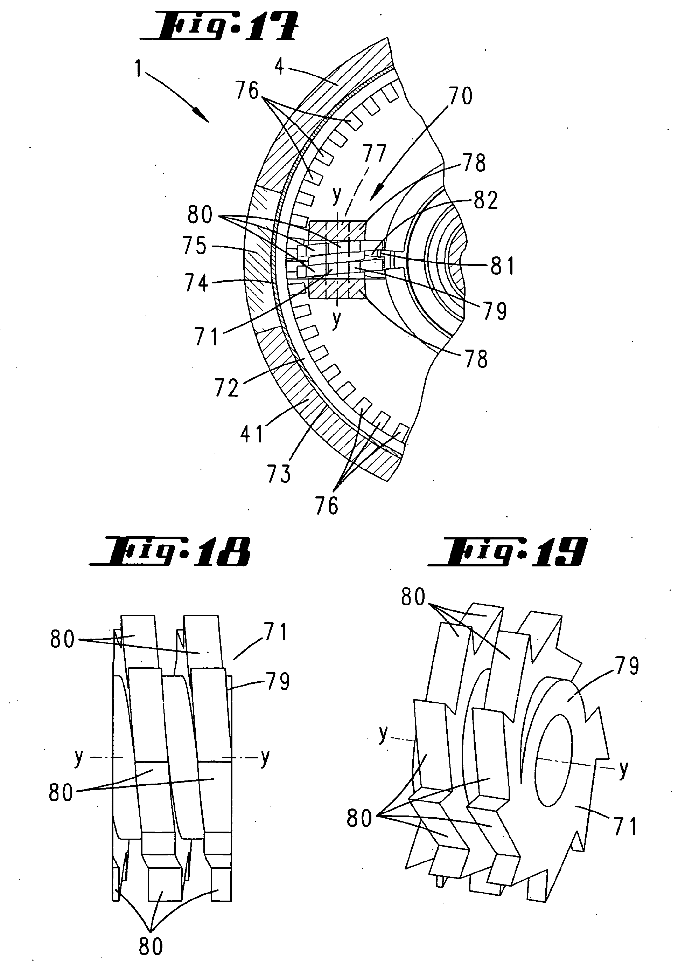

Die Figuren 13 bis 19 zeigen in einer weiteren Ausführungsform einen Inhalator 1, welcher zunächst gemäß der ersten Ausführungsform als Oral-Inhalator ausgebildet ist. Es ist ein Schrittschaltwerk 70 vorgesehen, zur Registrierung und Anzeige der durchgeführten Inhalationsvorgänge. Dieses Schrittschaltwerk 70 ist in der über das Gehäuse 2 vorstehenden Betätigungstaste 4 angeordnet, so weiter unmittelbar unterhalb der Decke der Betätigungstaste 4, die mit dem Mitnehmer 38 des Stabes 36 zusammenwirkenden Schlepparme 39 umgebend.

Das Schrittschaltwerk 70 besteht im wesentlichen aus einem Schrittschaltrad 71 und einem Skalenring 72, welcher mit dem Schrittschaltrad 71 in Formschlusseingriff steht.

Der Skalenring 72 ist manschettenartig unmittelbar unterseitig der Betätigungsdecke angeordnet, so weiter konzentrisch zur Gehäuseachse x. Die umlaufende Skalenringwandung liegt in einer Radialerweiterung der Wandung 41 der Betätigungstaste 4 ein, ist entsprechend in dieser Radialaufnahme 73 der Wandung 41 eine Drehung um die Gehäuseachse x zulassend gehaltert. Mantelaußenseitig ist der Skalenring 72 mit einer nicht näher dargestellten Skala 74 versehen, so beispielsweise mit einer solchen bedruckt. Sichtbar wird diese Skala 64 durch ein in einem Übergangsbereich von der Wandung 41 in die Decke der Betätigungstaste 4 wandungsseitig vorgesehenes Fenster 75.

Mantelinnenseitig des Skalenrings 72 ist dieser mittig der Vertikalerstreckung mit radial nach innen vorstehenden Mitnahmevorsprüngen 76 versehen. Es ist über den Umfang betrachtet unter Einhaltung gleichmäßiger Abstände zueinander eine Mehrzahl solcher Mitnahmevorsprünge 76 vorgesehen, so bspw. eine Anzahl, welche der maximalen Anzahl von Inhalationsauslösungen entspricht. In dem dargestellten Ausführungsbeispiel hingegen ist eine weitaus geringere Anzahl gewählt. Die Mitnahme des Skalenrings 72 über die Vorsprünge 76 erfolgt hier untersetzt.

Das Schrittschaltrad 71 wirkt formschlüssig mit diesen Mitnahmevorsprüngen 76 zusammen, wobei das Schrittschaltrad 71 um eine quer zur Gehäuseachse x ausgerichtete Drehachse y drehbeweglich ist, welche Achse y in der durch die Mitnahmevorsprünge 76 aufgespannten Ebene sich erstreckt. Konstruktiv ist die Achse y durch einen Achskörper 77 gelöst, der beidseitig endseitig in von der BetäNgungstastendecke nach innen ragend abhängenden Halterungsarmen 78 gefasst ist.

Das Schrittschaltrad 71 ist im wesentlichen als eingängige Schneckenwelle 79 ausgebildet mit einer Schneckenwendelsteigung, die angepasst ist an die Beabstandung zweier benachbarter Mitnahmevorsprünge 76 in Umfangsrichtung. Die Schneckenwelle 79 ist in Achsrichtung derselben betrachtet mit einer solchen Länge versehen, dass sich insgesamt im wesentlichen betrachtet zwei bis drei Schneckenwendelabschnitte ergeben. Der jeweilig zugeordnete Mitnahmevorsprung 76 tritt auf Achshöhe des Schrittschaltrades 71 zwischen die Flanken der Schneckenwendel ein. Bei Drehverlagerung der Schneckenwelle 79 um die Achse y erfolgt entsprechend eine Verlagerung des gefassten Mitnahmevorsprunges 76 in Horizontalrichtung, das heißt quer zur Gehäuseachse x.

Die umlaufende Stirn-Randkante der Schneckenwendel ist sägezahnartig profiliert, wobei jeder Zahn 80 eine steile, annähernd an einer Radialen orientierte Flanke und rückwärtig hierzu eine flach hin zum nachfolgenden Zahn 80 abfallende Flanke aufweist. In dem dargestellten Ausführungsbeispiel sind im Querschnitt durch das Schrittschaltrad 71 über den Umfang neun Zähne 80 ausgebildet, die weiter so bemessen sind, dass in Querbetrachtung, d. h. in Ausrichtung der Achse y betrachtet, die Zähne 80 eines Wendelabschnitts in horizontaler Projektion in Überdeckung zu den Zähnen 80 des benachbarten Wendelabschnitts liegen. Demzufolge sind auch die jeweiligen Zahnflanken benachbarter Wendelabschnitte in einer gemeinsamen Ebene ausgerichtet.

Die Zähne 80 wirken mit einem Zählfinger 81 zusammen, der parallel zur Drehachse y des Schrittschaltrades 71 betrachtet im freien Endbereich, das heißt in dem mit dem Schrittschaltrad 71 zusammenwirkenden Betätigungsendabschnitt 82 eine Breite aufweist, die in etwa der in selber Richtung gemessenen Breite des Schrittschaltrades 71 entspricht.

Der Zählfinger 81 wurzelt in dem die Arme 49 der Druckstücke 48 tragenden kreisringförmigen Träger und wächst stabartig unter Parallelausrichtung zur Gehäuseachse x nach oben in Richtung auf das in Grundstellung gemäß der Darstellung in Figur 13 vertikal beabstandete Schrittschaltrad 71 aus, dies unter endseitiger Ausformung des vorbeschriebenen, eine Spitze formenden Betätigungsabschnittes 82.

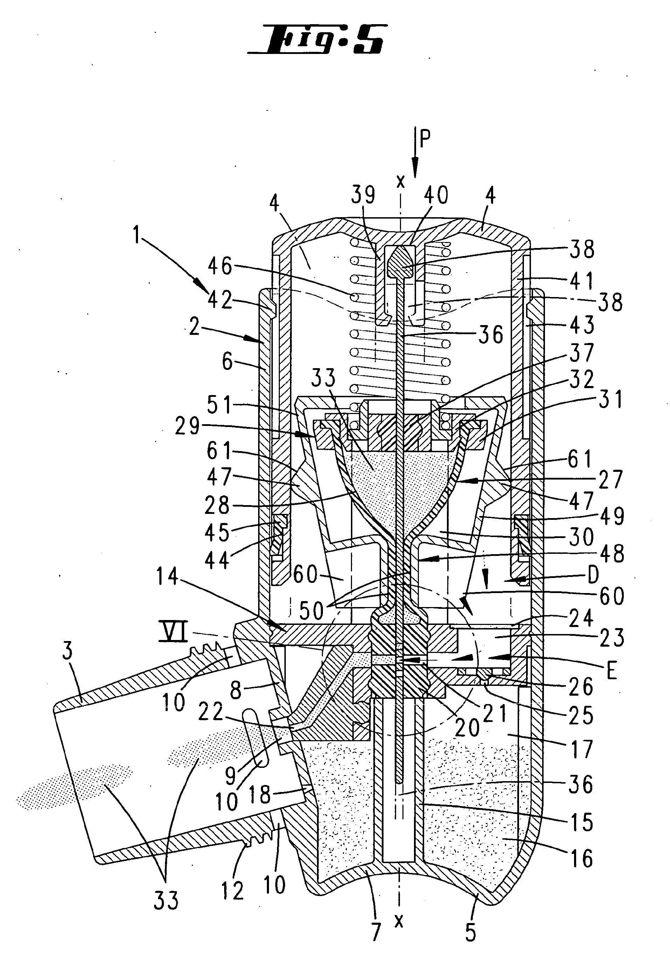

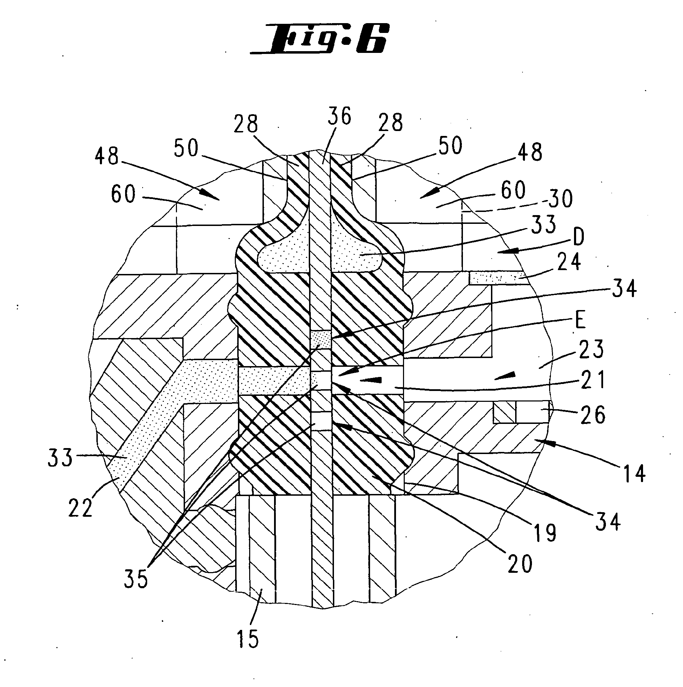

Der vertikale Abstand zwischen dem Betätigungsabschnitt 82 des Zählfingers 81 und der zu betätigenden steilen Flanke eines nach radial innen weisenden Zahns 80 des Schrittschaltrades 71 ist entsprechend dem Hub der Betätigungstaste 4 ausgehend von der Grundstellung in Figur 13 bis zur Übergabeposition des Medikaments aus der gefüllten Dosierkammer 34 in den Luftströmungskanal 21 gemäß der Darstellung in Figur 5 bzw. Figur 6 gewählt. Demzufolge wird das Schrittschaltwerk 70 erst dann betätigt, das heißt um eine Position weiter geschaltet, wenn im Zuge der Abwärtsverlagerung der Betätigungstaste 4 zunächst die Befüllung der Dosierkammern 34 über die Druckstücke 48 durchgeführt und hiernach unter weiterer Abwärtsverlagerung der Betätigungstaste 4 der zum schlagartigen Ausblasen der Dosierkammern 34 benötigte Ausblasluftdruck erreicht ist. Durch weitere Abwärtsverlagerung der Betätigungstaste 4 gelangen, wie beschrieben, die Dosierkammern 34 in den Bereich des Luftströmungskanals 21, durch welchen schlagartig infolge des aufgebauten Luftdruckes die Substanz ausgeblasen wird. Diese weitere vertikal abwärts gerichtete Betätigungsverlagerung der Taste 4 führt zu einer Drehverlagerung des Schrittschaltrades 71 um ein Zahnsegment. Die rückwärtig vorgesehenen flach ansteigenden Zahnflanken ermöglichen das Abgleiten des Betätigungsabschnittes 82 bei Rückverlagerung der Betätigungstaste 4 in die Grundstellung. Ein deckenseitig der Betätigungstaste 4 nach unten abragender, gegenüberliegend zum Betätigungsabschnitt 82 auf einen Zahn 80 einwirkender Rückhaltefinger 83 verhindert eine Rückdrehung des Schrittschaltrades 71 entgegen der vorgegebenen Zähl-Verlagerungsrichtung.

Der Inhalator kann verschiedene Arzneimittel und/oder bioaktive Substanzen zur Inhalation enthalten.

Als bioaktive Substanz kann ein beliebiger therapeutischer oder diagnostischer Wirkstoff gewählt werden, beispielsweise aus der Gruppe der Antiallergika, Bronchodilatatoren, Bronchokonstriktoren, Lungensurfaktantien, Analgetika, Antibiotika, Leukotrieninhibitoren oder -antagonisten, Anticholinergika, Mastzelleninhibitoren, Antihistaminika, Antiphlogistika, Antineoplastika, Anästhetika, Antituberkulotika, Kontrastmittel, kardiovaskuläre Wirkstoffe, Enzyme, Steroide, genetisches Material, Virenvektoren, Gegenstrang-Reagenzien, Proteine oder Peptide sowie Kombinationen dieser Substanzen.

Beispiele für spezifische Arzneimittel, mit denen der Inhalator gemäß Patentbeschreibung befüllt werden kann, sind u. a. Mometason, Ipratropiumbromid, Tiotropium und dessen Salze, Salmeterol, Fluticasonpropionat, Beclomethasondipropionat, Reproterol, Clenbuterol, Rofleponid und dessen Salze, Nedocromil, Natriumcromoglykat, Flunisolid, Budesonid, Formoterolfumaratdihydrat, Symbicort® (Budesonid und Formoterol), Terbutalin, Terbutalinsulfat, Salbutamolbase und -sulfat, Fenoterol, 3-[2-(4-Hydroxy-2-oxo-3H-1,3-benzothiazol-7-yl)ethylamin]-N-[2-[2-(4-methylphenyl)ethoxy]ethyl]propansulfonamid, Hydrochlorid. Alle vorgenannten Verbindungen können in freier Grundform oder als pharmazeutische Salze gemäß pharmazeutischer Praxis vorliegen.

Es können auch Arzneimittelkombinationen eingesetzt werden, so beispielsweise Formoterol/Budesonid; Formoterol/FluHcason; Formoterol/Mometason; Salmetcrol/Fluticason; Formoterol/Tiotropiumsalze; Zafirlukast/Formoterol, Zafirlukast/Budesonid; Montelukast/Formotcrol; Montelukast/Budesonid; Loratadin/Montelukast und Loratadin/Zafirlukast.

Weitere mögliche Kombinationen sind u. a. Tiotropium und Fluticason, Tiotropium und Budesonid, Tiotropium und Mometason, Mometason und Salmeterol, Formoterol und Rofleponid, Salmeterol und Budesonid, Salmeterol und Rofleponid sowie Tiotropium und Rofleponid.

## Patentansprüche

1. Inhalator (1) für pulverförmige Substanzen (33), insbesondere medizinische Substanzen, mit einer Substanzen-Vorratskammer (27) und einer eine bestimmte Menge davon aufnehmenden Dosierkammer (34), welche als Querbohrung (35) eines Stabes (36) ausgebildet und von einer Befüllungsstellung (B) in eine Entleerungsstellung (E) verschiebbar ist, in welcher Entleerungsstellung (E) die Dosierkammer (34) in einem Luftströmungskanal (21) liegt, **dadurch gekennzeichnet, dass** der Stab (36) mehrere am Stab (36) hintereinander liegende Dosierkammern (34) besitzt, die während einer einzelnen Ausgabebetätigung nacheinander in die Entleerungsstellung (E) treten und Stück für Stück von einem Luftüberdruck im Luftströmungskanal (21) ausblasbar sind.

2. Inhalator nach Anspruch 1, **gekennzeichnet durch** einen Leerhub zwischen einer Inhalator-Betätigungstaste (4) und Stab (36) derart, dass vor Mitnahme des Stabes (36) eine Einwölbung einer Vorratskammerwand (28) mittels äußerer Druckstücke (48) erfolgt.

3. Inhalator nach Anspruch 2, **dadurch gekennzeichnet, dass** der Stab (36) als Flach-Stab ausgebildet ist und die Druckstücke (48) mit Backen (60) ausgestattet sind, welche Stoßflächen (50) besitzen, die in völlig eingeschwenkter Stellung der Druckstücke (48) parallel zur Breitseiten-Wandfläche des Flach-Stabes (36) liegen.

4. Inhalator nach einem oder mehreren der Ansprüche 2 und 3, **dadurch gekennzeichnet, dass** die Betätigungstaste (4) kopfseitig dem Inhalatorgehäuse (2) vorsteht, entgegen einer Rückstellfeder (46) verlagerbarist, und nach kurzem Leerhub den Stab (36) vor sich herschiebt und über Auflaufschrägen (61) die Druckstücke (48) in Richtung des Stabes (36) schwenkt.

5. Inhalator nach einem oder mehreren der vorhergehenden Ansprüche 2 bis 4, **dadurch gekennzeichnet, dass** sich die aus elastischem Material bestehende Vorratskammerwand (28) bei Betätigung des Inhalators (1) in Richtung des Stabes (36) mindestens im Dosierlochbereich bis zur Anlage am Stab (36) einwölbt.

6. Inhalator nach einem oder mehreren der vorhergehenden Ansprüche 2 bis 5, **dadurch gekennzeichnet, dass** sich bei Verlagerung der Betätigungstaste (4) im Inhalatorgehäuse (2) ein Luft-Überdruck aufbaut, der sich ebi Verlagerung des Stabes (36) in den Luftströmungskanal (21) zum Ausblasen der Substanz (33) fortpflanzt.

7. Inhalator nach einem oder mehreren der vorhergehenden Ansprüche 2 bis 6, **gekennzeichnet durch** einen elastischen Kolbenring (45) am einwärts liegenden Endbereich einer topfförmigen Wand (41) der Betätigungstaste (4), wobei das Außengehäuse des Inhalators (2) mit seiner Innenwand den Druckzylinder (D) für diesen Kolbenring (45) bildet.

8. Inhalator nach einem oder mehreren der vorhergehenden Ansprüche, **dadurch gekennzeichnet, dass** der Querschnitt des Stabes (36) einen Luftauslass verschließt.

9. Inhalator nach einem oder mehreren der vorhergehenden Ansprüche, **gekennzeichnet durch** eine Einstellvorrichtung (52) zur Bestimmung der Anzahl der in die Entleerungsstellung (E) bringbaren Dosierkammern (34).

10. Inhalator nach einem oder mehreren der vorhergehenden Ansprüche 2 bis 9, **gekennzeichnet durch** einen Einstellring (62) mit mehreren Stufen zum Stoppen der Verschiebebewegung der Betätigungstaste (4) nach Durchfahren einer bestimmten Anzahl von Dosierkammern (34) **durch** die Entleerungsstellung (E).

11. Inhalator nach einem oder mehreren der vorhergehenden Ansprüche, **dadurch gekennzeichnet, dass** der geradlinig quergerichtete Luftströmungskanal (21) sich in einem querschnittskleineren, schräg aufwärts gerichteten Endkanalabschnitt (67) fortsetzt, der innerhalb einer Nasentülle (65) rückversetzt zu deren freien Ende (69) endet.

12. Inhalator nach einem oder mehreren der vorhergehenden Ansprüche, **gekennzeichnet durch** ein bei Erreichen des Ausblasluftdruckes betätigtes Schrittschaltwerk (70).

13. Inhalator nach Anspruch 12, **gekennzeichnet durch** einen auf das Schrittschaltwerk (70) einwirkenden Zählfinger (81), dessen Spitze (82), ein Schrittschaltrad (71) erreicht etwa bei Beginn der Verlagerung des Stabes (36) mit den gefüllten Dosierkammern (34) in den Bereich des Luftströmungskanals (21).

14. Inhalator nach Anspruch 13 **dadurch gekennzeichnet, dass** das Schrittschaltrad (71) einen Skalenring (72) des Schrittschaltwerkes (70) schneckenradartig dreht.

## Claims

1. Inhaler (1) for powdery substances (33), in particular medical substances, with a substance storage chamber (27) and a dosing chamber (34), which receives a defined amount of the substance, is formed as a cross-hole (35) of a rod (36) and can be displaced from a filling position (B) into an emptying position (E), in which emptying position (E) the dosing chamber (34) is located in an air flow channel (21), **characterized in that** the rod (36) has a number of dosing chambers (34) that are located one after the other on the rod (36), which move into the emptying position (E) one after the other during an individual discharge actuation and can be blown out one by one by positive air pressure in the air flow channel (21).

2. Inhaler according to Claim 1, **characterized by** an inactive displacement between an inhaler actuating button (4) and the rod (36) with the effect that an inward curving of a storage chamber wall (28) by means of outer thrust pieces (48) takes place before the rod (36) is taken along.

3. Inhaler according to Claim 2, **characterized in that** the rod (36) is formed as a flat rod and the thrust pieces (48) are provided with jaws (60), which have abutting faces (50) that lie parallel to the wide-side wall surface of the flat rod (36) in the fully pivoted-in position of the thrust pieces (48).

4. Inhaler according to one or both of Claims 2 and 3, **characterized in that** the head of the actuating button (4) protrudes above the inhaler housing (2), can be displaced against a return spring (46) and, after a short inactive displacement, pushes the rod (36) before it and pivots the thrust pieces (48) in the direction of the rod (36) by means of run-on slopes (61).

5. Inhaler according to one or more of the preceding Claims 2 to 4, **characterized in that**, when the inhaler (1) is actuated, the storage chamber wall (28) consisting of elastic material is made to curve in the direction of the rod (36), at least in the region of the dosing holes, until it abuts against the rod (36).

6. Inhaler according to one or more of the preceding Claims 2 to 5, **characterized in that**, with displacement of the actuating button (4) in the inhaler housing (2), a positive air pressure is built up, which proceeds to blow out the substance (33) by displacement of the rod (36) into the air flow channel (21).

7. Inhaler according to one or more of the preceding Claims 2 to 6, **characterized by** an elastic piston ring (45) at the inward end region of a cup-shaped wall (41) of the actuating button (4), the outer housing of the inhaler (2) forming with its inner wall the pressure cylinder (D) for this piston ring (45).

8. Inhaler according to one or more of the preceding claims, **characterized in that** the cross-section of the rod (36) closes an air outlet.

9. Inhaler according to one or more of the preceding claims, **characterized by** a setting device (52) for determining the number of dosing chambers (34) that can be brought into the emptying position (E).

10. Inhaler according to one or more of the preceding Claims 2 to 9, **characterized by** a setting ring (62) with a number of steps for stopping the displacing movement of the actuating button (4) after a specific number of dosing chambers (34) have passed through the emptying position (E).

11. Inhaler according to one or more of the preceding claims, **characterized in that** the air flow channel (21) directed straight across continues into an obliquely upwardly directed end channel portion (67) of a smaller cross-section, which ends within a nose tube (65) such that it is set back from the free end (69) of the latter.

12. Inhaler according to one or more of the preceding claims, **characterized by** an indexing mechanism (70) that is actuated when the blowing-out air pressure is reached.

13. Inhaler according to Claim 12, **characterized by** a counting finger (81) acting on the indexing mechanism (70), the tip (82) of which finger reaches an indexing wheel (71) approximately at the beginning of the displacement of the rod (36) with the filled dosing chambers (34) into the region of the air flow channel (21).

14. Inhaler according to Claim 13, **characterized in that** the indexing wheel (71) turns a scale ring (72) of the indexing mechanism (70) in the manner of a worm wheel.

## Revendications

1. Inhalateur (1) pour substances (33) pulvérulentes, en particulier substances médicinales, avec une chambre de stockage de substance (27) et une chambre de dosage (34), recevant une quantité déterminée de celles-ci, réalisée sous la forme de perçage transversal (35) d'une barre (36), et susceptible d'être déplacée d'une position de remplissage (B) en une position de vidage (E), position de vidage (E) dans laquelle la chambre de dosage (34) est située dans un canal d'écoulement d'air (21), **caractérisé en ce que** la barre (36) comprend plusieurs chambres de dosage (34), situées les unes derrière les autres sur la barre (36), qui, pendant un actionnement de distribution individuel, passent les unes après les autres à la position de vidage (E) et sont susceptibles d'être soumises à une chasse par soufflage, une par une, par une surpression d'air dans le canal d'écoulement d'air (21).

2. Inhalateur selon la revendication 1, **caractérisé par** une course à vide, entre une touche d'actionnement d'inhalateur (4) et la barre (36), de manière que, avant l'entraînement de la barre (36), une incurvation d'une paroi de chambre de stockage (28) se produise au moyen de pièces de pressage (48) extérieures.

3. Inhalateur selon la revendication 2, **caractérisé en ce que** la barre (36) est réalisée sous la forme de barre plate, et les pièces de pressage (48) sont équipées de mâchoires (60) comprenant des faces de joint (50) qui, lorsque les pièces de pressage (48) sont complètement escamotées par pivotement, sont situées parallèlement à la surface de paroi de côté large de la barre plate (36).

4. Inhalateur selon une ou plusieurs des revendications 2 et 3, **caractérisé en ce que** la touche d'actionnement (4) fait saillie, côté tête, du boîtier d'inhalateur (2), est déplaçable à l'encontre d'un ressort de rappel (46) et, après une courte course à vide, ramène par poussée devant soi la barre (36), et fait pivoter, sur des chanfreins de franchissement (61), les pièces de pressage (48) en direction de la barre (36).

5. Inhalateur selon une ou plusieurs des revendications 2 à 4 précédentes, **caractérisé en ce que**, lors de l'actionnement de l'inhalateur (1), la paroi de chambre de stockage (28), composée de matériau élastique, s'incurve en direction de la barre (36), au moins dans la zone de trou de dosage, jusqu'à venir en appui sur la barre (36).

6. Inhalateur selon une ou plusieurs des revendications 2 à 5 précédentes, **caractérisé en ce que**, en cas de déplacement de la touche d'actionnement (4), dans le boîtier d'inhalateur (2) s'établit une surpression d'air qui, lors du déplacement de la barre (36), se propage dans le canal d'écoulement d'air (21), pour aboutir à la chasse par soufflage de la substance (33).

7. Inhalateur selon une ou plusieurs des revendications 2 à 6 précédentes, **caractérisé par** un segment de piston (45) élastique, sur la zone d'extrémité située intérieurement, d'une paroi (41), en forme de pot, de la touche d'actionnement (4), le boîtier extérieur de l'inhalateur (2) formant, avec sa paroi intérieure, le cylindre à pression (D) pour ce segment de piston (45).

8. Inhalateur selon une ou plusieurs des revendications précédentes, **caractérisé en ce que** la section transversale de la barre (36) obture un échappement d'air.

9. Inhalateur selon une ou plusieurs des revendications précédentes, **caractérisé par** un dispositif de réglage (52), pour déterminer le nombre des chambres de dosage (34) susceptibles d'être placées à la position de vidage (E).

10. Inhalateur selon une ou plusieurs des revendications 2 à 9 précédentes, **caractérisé par** une bague de réglage (62), avec plusieurs degrés pour stopper le déplacement de la touche d'actionnement (4) après passage d'un nombre déterminé de chambres de dosage (34) par la position de vidage (E).

11. Inhalateur selon une ou plusieurs des revendications précédentes, **caractérisé en ce que** le canal d'écoulement d'air (21), orienté transversalement et rectiligne, se prolonge en un tronçon de canal d'extrémité (67) à plus petite section transversale, orienté obliquement vers le haut, qui s'achève à l'intérieur d'un bec de nez (65), en retrait de son extrémité (69) libre.

12. Inhalateur selon une ou plusieurs des revendications précédentes, **caractérisé par** un mécanisme de commutation pas à pas (70), actionné à l'atteinte de la pression d'air de soufflage.

13. Inhalateur selon la revendication 12, **caractérisé par** un doigt de comptage (81), agissant sur le mécanisme de commutation pas à pas (70), dont la pointe (82) atteint une roue de commutation pas-à-pas (71) à peu près au début du déplacement de la barre (36) avec les chambres de dosage (34) remplies, dans la zone du canal d'écoulement d'air (21).

14. Inhalateur selon la revendication 13, **caractérisé en ce que** la roue de commutation pas-à-pas (71) fait tourner, à la manière d'une roue à vis, un anneau à échelle (72) du mécanisme de commutation pas à pas (70).
